# EUROPEAN PATENT APPLICATION

(11) **EP 4 681 664 A1**
(43) Date of publication of application: **21.01.2026**
(21) Application number: 25186921.0
(22) Date of filing: 02.07.2025
(51) Int. Cl.: A61B 17/86, A61B 17/88, A61C 8/00

(54) **DUAL INTERFACE SCREW ELEMENT AND SYSTEM**

(30) Priority: 15.07.2024 US 202418772435
(71) Applicant: Unger, Yehonathan, 2521300 Moshav Bustan HaGalil (IL)
(72) Inventor: Unger, Yehonathan, 2521300 Moshav Bustan HaGalil (IL)
(74) Representative: Kancelaria Eupatent.pl Sp. z o.o.

(57) **Abstract**

A dual interface screw element includes a screw body (10) with an external thread (12) and two distinct engagement interfaces (13, 15), one for inserting the screw and another for removing it. The removal engagement interface (15) includes a reverse thread (16) located in an axial recess (36) such that a complementary reverse-threaded tool (24) applying torque to extract the screw body is tightened against the second engagement interface. The screw is part of a system including an extraction tool (24) and an insertion tool (18). Applications include medical applications such as dental implants and bone screws, domestic applications such as headless wall mounting anchors, and a wide range of other industrial and aerospace applications.

## Description

### FIELD AND BACKGROUND OF THE INVENTION

The present invention relates to screws and, in particular, it concerns a screw element and corresponding system with distinct interfaces for inserting and extracting the screw.

Screws are among the most ubiquitous mechanical elements, employed for an unlimited range of applications ranging from medical devices to heavy machinery and from household appliances to aerospace applications.

There are numerous types of screw heads designed to interface with different screwdrivers or driving tools. Common examples include Phillips, flat head, Robertson (square), and Torx. Different head designs offer different advantages and disadvantages, each with its own limitations on what maximum torque can be applied.

A problem common to all screw interfaces is that the screw may disengage or fall from the driver while trying to position it for insertion. This can in some cases be ameliorated by employing a magnetic bit, but his still allows some degree of misalignment between the driver and the screw which may hamper insertion and does not provide a solution for non-magnetic materials.

A further problem relates to unscrewing to remove a screw. In this case, there is an inherent conflict between the strong forward axial force which is required to maintain reliable engagement of a driver with the screw head and the desired direction of motion of the screw which would be enhanced by a rearward extraction force. Furthermore, depending on the materials of the screw and the surrounding material, and on how long the screw has been in place, the torque required for extraction may be larger than the insertion torque, sometimes exceeding the torque which can be delivered through the screw interface.

### SUMMARY OF THE INVENTION

The present invention is a screw element and corresponding system with distinct interfaces for inserting and extracting the screw.

According to the teachings of an embodiment of the present invention there is provided, a dual interface screw element comprising: (a) a screw body having a central axis, a proximal end, a distal end and a length, the screw body having an external thread along at least part of the length; (b) a first engagement interface accessible from the proximal end of the screw body for engagement to supply torque in an insertion direction of the external thread; and (c) a second engagement interface located in an axial recess accessible from the proximal end of the screw body, the second engagement interface including a reverse thread such that a complementary reverse-threaded tool applying torque to extract the screw body is tightened against the second engagement interface.

According to a further feature of an embodiment of the present invention, the external thread is a right-handed thread and wherein the reverse thread is a left-handed thread.

According to a further feature of an embodiment of the present invention, the first engagement interface includes a thread located within the axial recess.

According to a further feature of an embodiment of the present invention, the thread of the first engagement interface is closer to the proximal end than the reverse thread of the second engagement interface.

According to a further feature of an embodiment of the present invention, the thread of the first engagement interface is further from the proximal end than the reverse thread of the second engagement interface.

There is also provided according to the teachings of an embodiment of the present invention, a screw system comprising: (a) the aforementioned screw element; and (b) an extraction tool comprising a reverse-threaded portion configured for insertion into the axial recess and for engaging the second engagement interface.

There is also provided according to the teachings of an embodiment of the present invention, a screw system comprising: (a) the aforementioned screw element; (b) an insertion tool comprising a threaded portion configured for insertion into the axial recess and for engaging the first engagement interface; and (c) an extraction tool comprising a reverse-threaded portion configured for insertion into the axial recess and for engaging the second engagement interface.

According to a further feature of an embodiment of the present invention, the screw body is a dental implant.

According to a further feature of an embodiment of the present invention, the screw body is a bone screw.

According to a further feature of an embodiment of the present invention, the screw body is a bolt forming part of a joint between two structural elements.

There is also provided according to the teachings of an embodiment of the present invention, a dual interface screw element comprising: (a) a screw body having a central axis, a proximal end, a distal end and a length, the screw body having an external thread along at least part of the length; (b) a first engagement interface accessible from the proximal end of the screw body for engagement to supply torque in an insertion direction of the external thread; and (c) a second engagement interface located in an axial recess accessible from the proximal end of the screw body, the second engagement interface including a reverse helical engagement configuration such that a complementary reverse-helical engagement tool applying torque to extract the screw body is tightened against the second engagement interface.

According to a further feature of an embodiment of the present invention, the reverse helical engagement configuration is a reverse screw thread.

According to a further feature of an embodiment of the present invention, the reverse helical engagement configuration is a helical socket of hexagonal cross-section.

According to a further feature of an embodiment of the present invention, the reverse helical engagement configuration is a helical socket of star cross-section.

### BRIEF DESCRIPTION OF THE DRAWINGS

The invention is herein described, by way of example only, with reference to the accompanying drawings, wherein:
FIG. 1 is a schematic isometric view of a screw element constructed and operative according to an embodiment of the present invention;
FIG. 2 is an axial cross-sectional view of the screw element of FIG. 1 revealing dual engagement interfaces;
FIG. 3 is a schematic isometric view of an insertion tool for engaging a first engagement interface of the screw element of FIG. 1;
FIG. 4 is a schematic isometric view of a removal tool for engaging a second engagement interface of the screw element of FIG. 1;
FIG. 5 is an axial cross-sectional view showing the insertion tool of FIG. 3 engaged with the screw element of FIG. 1;
FIG. 6 is an axial cross-sectional view showing the removal tool of FIG. 4 engaged with the screw element of FIG. 1;
FIG. 7 is an axial cross-sectional view of a variant implementation of the present invention for use as a dental implant;
FIG. 8 is an axial cross-sectional view of a further variant implementation of the present invention for use as a bone screw;
FIG. 9 is a view similar to FIG. 8 illustrating an alternative implementation of the first engagement interface as a hex socket;
FIG. 10 is a view similar to FIG. 8 illustrating an alternative implementation in which the axial positions of the first and second engagement interfaces are exchanged;
FIG. 11 is a view similar to FIG. 10 illustrating an alternative implementation of the first engagement interface as a hex socket;
FIG. 12 is an isometric view cut-away along an axial plane illustrating an alternative implementation of the screw element of FIG. 1 employing helical engagement configurations with a hexagonal cross-section;
FIGS. 13 and 14 are partial isometric views of a complementary insertion tool and a complementary removal tool, respectively, for use with the screw element of FIG. 12;
FIGS. 15A and 15B are isometric views illustrating a further alternative implementation of the screw element of FIG. 1 employing helical engagement configurations with a star cross-section, shown, respectively, in full view and cut-away along an axial plane; and
FIGS. 16 and 17 are partial isometric views of a complementary insertion tool and a complementary removal tool, respectively, for use with the screw element of FIG. 15A.

### DESCRIPTION OF THE PREFERRED EMBODIMENTS

The present invention is a screw element and corresponding system with distinct interfaces for inserting and extracting the screw.

The principles and operation of screw elements and systems according to the present invention may be better understood with reference to the drawings and the accompanying description.

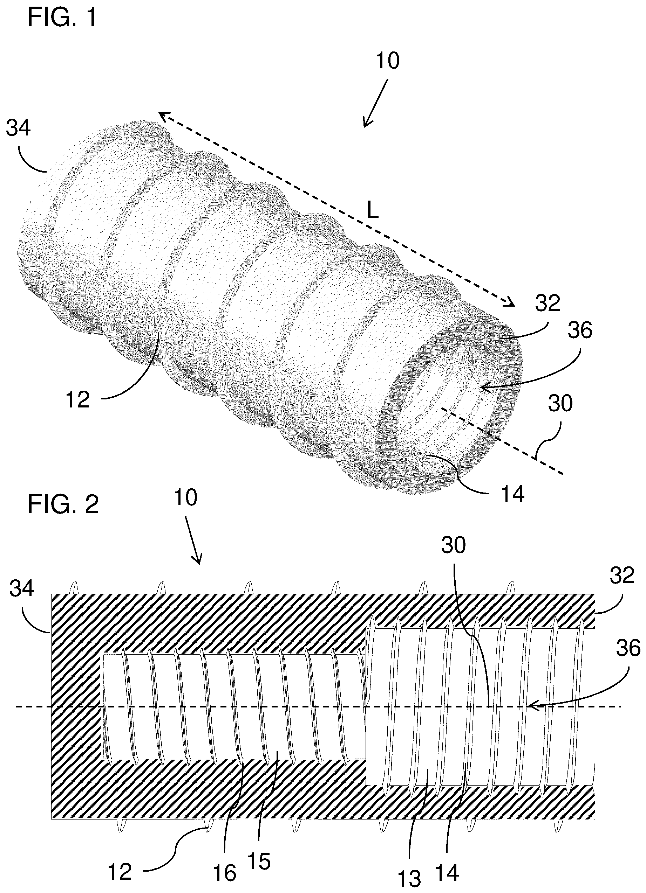

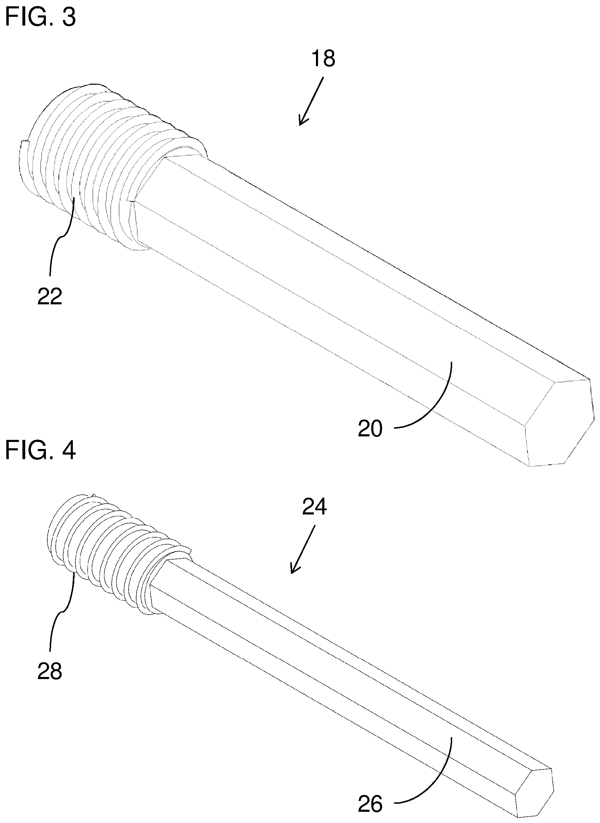

Referring now to the drawings, FIGS. 1 and 2 show schematically a dual interface screw element, constructed and operative according to an embodiment of the present invention. In general terms, the screw element has a screw body **10** having a central axis **30,** a proximal end **32,** a distal end **34** and a length **L.** Screw body **10** has an external thread **12** along at least part of the length. The screw element has two distinct engagement interfaces, one designed for use while inserting the screw and another for use when removing (unscrewing) the screw. Thus, a first engagement interface **13,** accessible from the proximal end of the screw body, is configured for engagement to supply torque in an insertion direction of the external thread. In the non-limiting but preferred case illustrated here, this first engagement interface **13** includes a thread **14** located within an axial recess **36.** A second engagement interface **15,** located in axial recess **36** accessible from the proximal end **32** of screw body **10,** includes a reverse thread **16** such that a complementary reverse-threaded tool (FIG. 4, below) applying torque to extract the screw body is tightened against the second engagement interface. In the case of a conventional right-handed external thread **12,** the "reverse" thread **16** is a left-handed thread. In any special application requiring a left-handed external thread 12, the "reverse" thread **16** would be a right-handed thread.

The screw of FIG. 1 is preferably provided as part of a screw system which includes an extraction tool **24** (FIG. 4), having a reverse-threaded portion **28** configured for insertion into the axial recess **36** to engage thread **16** of the second engagement interface **15.** The screw system preferably also includes an insertion tool **18** (FIG. 3) having a threaded portion **22** configured for insertion into axial recess **36** to engage thread **14** of the first engagement interface **13.** Insertion tool **18** and extraction tool **24** each has a shaft **20** and **26,** respectively, for delivering torque to the screw during insertion or extraction. In the non-limiting but preferred case illustrated here, these shafts are hexagonal shafts suitable for mounting in a conventional powered drill-driver or impact driver. Optionally, both tool ends (threaded portions **22** and **28**) may be incorporated on opposite ends of a single straight hex shaft to form a reversible tool (not shown), or on opposite ends of an L-shaped shaft to form a manual tool for insertion and extraction that is operated similarly to an Allen key. Alternative implementations may employ a manually operated handle on the shaft.

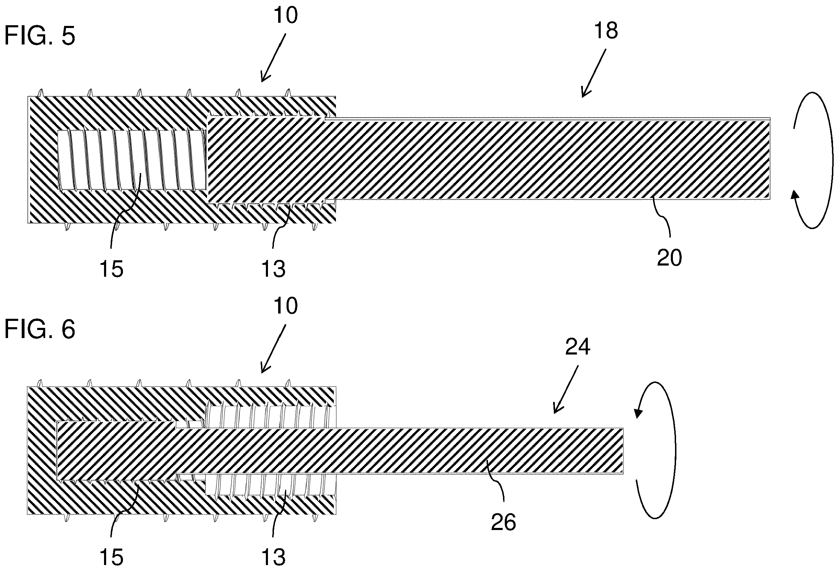

FIG. 5 illustrates schematically use of insertion tool **18** to insert screw body **10** into a host material (not shown). Threaded portion **22** engages thread **14** within the proximal part of axial recess **36** through a right-handed rotation until it is fully inserted, which may be defined by either a distal part of shaft **20** abutting proximal end **32** of the screw body or by the threaded portion **22** abutting an internal shoulder of recess **36,** as illustrated in FIG. 5. In this state, the screw body is firmly gripped and aligned with the insertion tool, preventing accidental dropping or misalignment of the screw. Further right-handed rotation of insertion tool **18** transfers torque to screw body **10** so as to insert the screw body into the host material in a conventional manner.

FIG. 6 illustrates schematically use of extraction tool **24** to extract (unscrew) screw body **10** from a host material (not shown). Threaded portion **28** is inserted through the proximal part of axial recess **36,** being undersized so as not to interact with thread **14,** and then engages internal thread **16** through a left-handed rotation until fully engaged. The fully engaged state may be defined by a suitably positioned and sized shoulder formed on shaft **26** to engage the internal shoulder within recess **36** at the transition between the two engagement configurations, by a larger shoulder configured to abut proximal end **32** of the screw body, or by threaded portion **28** reaching the end of the threaded recess as shown in FIG. 6. In this state, the screw body is firmly gripped by the extraction tool so that further left-handed rotation of extraction tool **24** transfers torque to screw body **10** so as to unscrew the screw body into the host material. The reverse-threaded engagement ensures that the torque applied to unscrew the screw body only tightens engagement of extraction tool **24,** and this engagement also facilitates application of axial forces to aid withdrawal of the screw body from its host material.

Optionally, when engaging the smaller diameter engagement interface **15** further from the proximal end of the screw body, a cylindrical spacer element may be introduced in order to provide support for the part of the shaft **26** (or **20** if the interfaces are reversed, as discussed below) which is within the larger diameter part of the recess. In this case, the spacer element is preferably sized to contact the crests of the threads in the larger diameter part of the recess without engaging the threads.

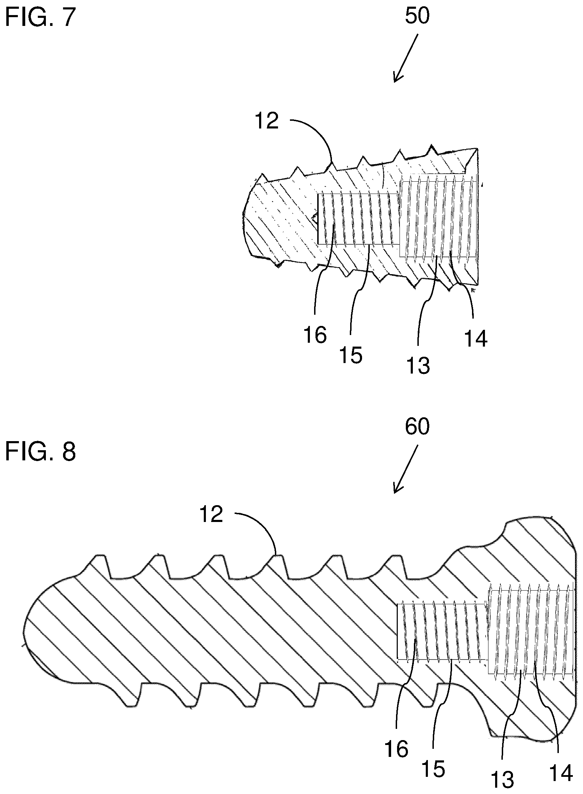

The screw element of FIG. 1 is illustrated only schematically as a cylindrical screw element with an external thread. The present invention may be used to advantage in all fields of application and all types of screw or bolt structures, particularly where large closing torque is required, and where subsequent extraction of a screw may otherwise be challenging. Non-limiting exemplary applications include implementations as a dental implant **50** (FIG. 7) or as a bone screw **60** (FIG. 8). Other applications include domestic and other applications such as, for example, as a headless wall anchor (in which case an object may be supported by engaging screw thread **14** of axial recess **36**) and in all sorts of screws and bolts for forming part of a joint between two structural elements, in domestic, industrial and aerospace applications.

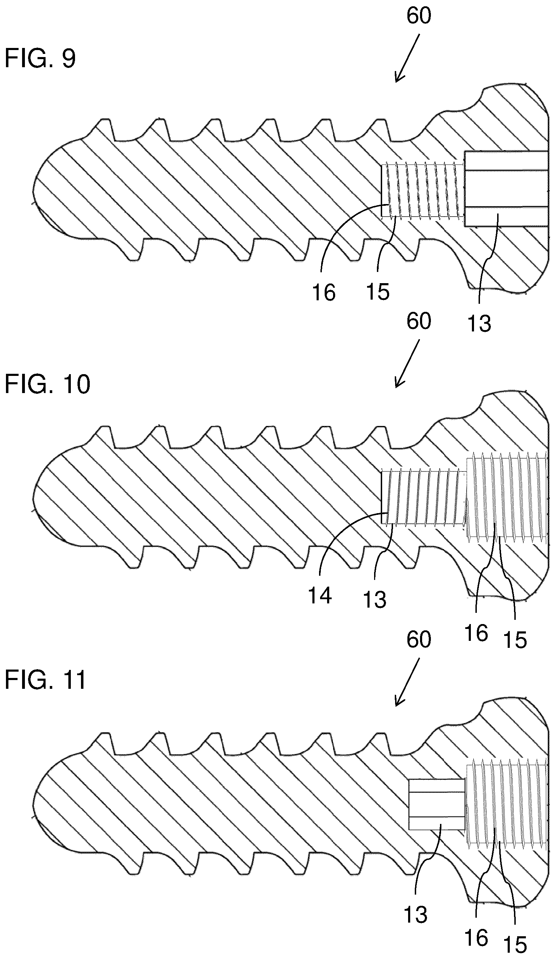

Although it is believed to be highly advantageous to implement both first engagement interface **13** and second engagement interface **15** as threaded interfaces (or, more generically, "helical engagement configurations" as discussed further below), it should be noted that screws and screw systems in which the first engagement interface is an otherwise conventional engagement interface also fall within the scope of the present invention. Thus, for example, the first engagement interface may be implemented in recess **36** and/or on the surface of the proximal end **32** of the screw as a straight slot, a Philips crosshead, a Torx star socket, a hex socket or any other conventional or non-standard screw interface. By way of one non-limiting example, FIG. 9 illustrates an exemplary embodiment in which first engagement interface **13** is implemented as a hex socket.

The exemplary implementations of the present invention illustrated thus far all have first engagement interface **13** closer to proximal end **32** than second engagement interface **15.** Depending on various design considerations, and most notably, the levels of torque for which the screw system is designed during the insertion and extraction processes, the interfaces may be reversed so that thread **14** of first engagement interface 13 is further from the proximal end **32** than reverse thread **16** of second engagement interface **15.** This option is illustrated in FIG. 10. A further variant implementation, generally similar to FIG. 10 but in which first engagement interface **13** is implemented as a hex socket, is illustrated in FIG. 11.

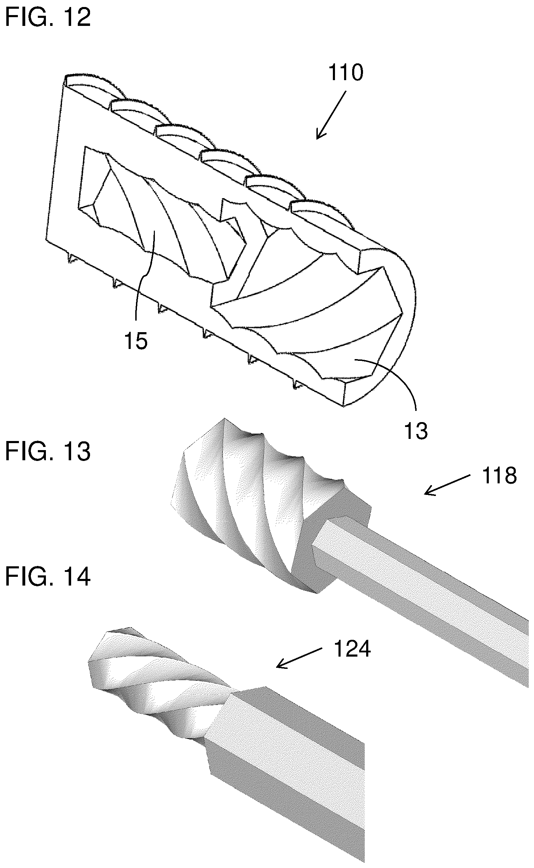

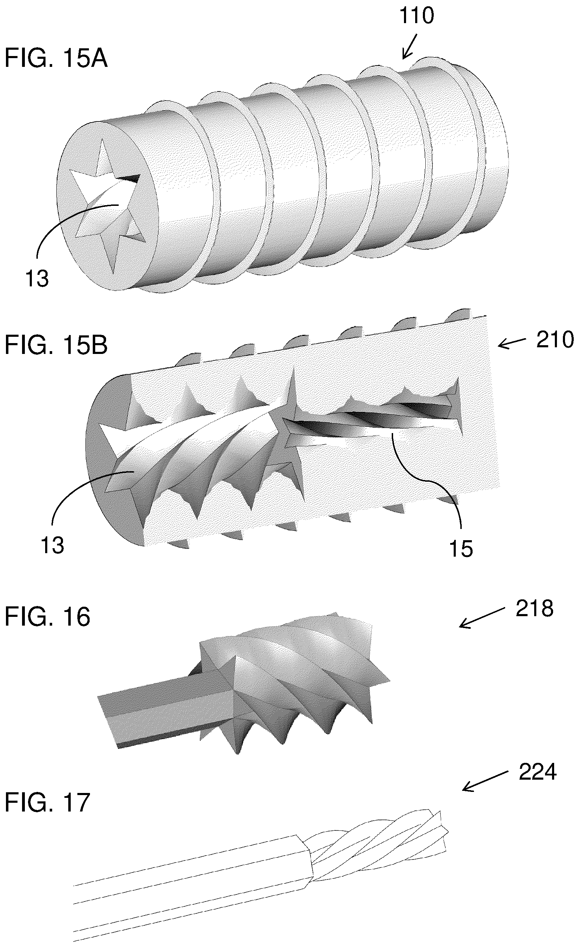

The threaded engagement of first and second engagement interfaces illustrated thus far are examples of a "helical engagement configuration," i.e., any complementary male-female engagement configuration in which engagement is achieved by a combination of simultaneous motion along an axis combined with rotation about that axis. Depending on details of the intended application, material properties and manufacturing capabilities, other forms of helical engagement configuration may be preferred for one or both of first engagement interface **13** and second engagement interface **15,** and the complementary insertion and extraction tools. By way of non-limiting examples, FIGS. 12-14 illustrate an embodiment of the screw element **110,** insertion tool **118** and extraction tool **124,** respectively, where each engagement interface is implemented as a helical engagement element which has a hexagonal cross-section perpendicular to its central axis at each point, and FIGS. 15A-17 illustrate an embodiment of the screw element **210,** insertion tool **218** and extraction tool **224,** respectively, where each engagement interface is implemented as a helical engagement element which has a star cross-section perpendicular to its central axis at each point. Here too, the various engagement interfaces may be of different types, the inner and outer configurations may be swapped axially, and first engagement interface may be replaced with an otherwise conventional interface.

It will be appreciated that the above descriptions are intended only to serve as examples, and that many other embodiments are possible within the scope of the present invention as defined in the appended claims.

## Claims

1. A dual interface screw element comprising:
(a) a screw body having a central axis, a proximal end, a distal end and a length, said screw body having an external thread along at least part of the length;
(b) a first engagement interface accessible from said proximal end of said screw body for engagement to supply torque in an insertion direction of said external thread; and
(c) a second engagement interface located in an axial recess accessible from said proximal end of said screw body, said second engagement interface including a reverse thread such that a complementary reverse-threaded tool applying torque to extract said screw body is tightened against said second engagement interface.

2. The screw element of claim 1, wherein said external thread is a right-handed thread and wherein said reverse thread is a left-handed thread.

3. The screw element of claim 1, wherein said first engagement interface includes a thread located within said axial recess.

4. The screw element of claim 3, wherein said thread of said first engagement interface is closer to said proximal end than said reverse thread of said second engagement interface.

5. The screw element of claim 3, wherein said thread of said first engagement interface is further from said proximal end than said reverse thread of said second engagement interface.

6. A screw system comprising:
(a) the screw element of claim 1; and
(b) an extraction tool comprising a reverse-threaded portion configured for insertion into said axial recess and for engaging said second engagement interface.

7. A screw system comprising:
(a) the screw element of claim 3;
(b) an insertion tool comprising a threaded portion configured for insertion into said axial recess and for engaging said first engagement interface; and
(c) an extraction tool comprising a reverse-threaded portion configured for insertion into said axial recess and for engaging said second engagement interface.

8. The screw element of claim 1, wherein said screw body is a dental implant.

9. The screw element of claim 1, wherein said screw body is a bone screw.

10. The screw element of claim 1, wherein said screw body is a bolt forming part of a joint between two structural elements.

11. A dual interface screw element comprising:
(a) a screw body having a central axis, a proximal end, a distal end and a length, said screw body having an external thread along at least part of the length;
(b) a first engagement interface accessible from said proximal end of said screw body for engagement to supply torque in an insertion direction of said external thread; and
(c) a second engagement interface located in an axial recess accessible from said proximal end of said screw body, said second engagement interface including a reverse helical engagement configuration such that a complementary reverse-helical engagement tool applying torque to extract said screw body is tightened against said second engagement interface.

12. The screw element of claim 11, wherein said reverse helical engagement configuration is a reverse screw thread.

13. The screw element of claim 11, wherein said reverse helical engagement configuration is a helical socket of hexagonal cross-section.

14. The screw element of claim 11, wherein said reverse helical engagement configuration is a helical socket of star cross-section.
